# EUROPÄISCHE PATENTSCHRIFT

(11) **EP 3 818 366 B1**
(45) Veröffentlichungstag und Bekanntmachung des Hinweises auf die Patenterteilung: **20.09.2023**
(21) Anmeldenummer: 19735244.6
(22) Anmeldetag: 26.06.2019
(51) Int. Cl.: G01N 27/417, G01N 33/00

(54) **VERFAHREN ZUM BETREIBEN EINES SENSORS ZUM NACHWEIS MINDESTENS EINES ANTEILS EINER MESSGASKOMPONENTE MIT GEBUNDENEM SAUERSTOFF IN EINEM MESSGAS**
METHOD FOR OPERATING A SENSOR FOR DETECTING AT LEAST A PORTION OF A MEASUREMENT GAS COMPONENT HAVING BOUND OXYGEN IN A MEASUREMENT GAS
PROCÉDÉ POUR FAIRE FONCTIONNER UN CAPTEUR DESTINÉ À DÉTECTER AU MOINS UNE FRACTION D'UN COMPOSANT D'UN GAZ DE MESURE AVEC DE L'OXYGÈNE LIÉ DANS UN GAZ DE MESURE

(30) Priorität: 02.07.2018 DE 102018210846
(43) Veröffentlichungstag der Anmeldung: 12.05.2021
(73) Patentinhaber: Robert Bosch GmbH, 70442 Stuttgart (DE)
(72) Erfinder: OECHTERING, Peter, 76229 Karlsruhe (DE); HEISE, Michael, 01445 Radebeul (DE); GUEL, Mustafa, 71522 Backnang (DE); FIEDLER, Michael, 71034 Boeblingen (DE); SCHROEDER, Andy, 5405 Daettwil (CH); SINGER, Matthias, 73730 Esslingen (DE); DAECKE, Dirk, 70619 Stuttgart (DE)
(86) Internationale Anmeldenummer: PCT/EP2019/066943
(87) Internationale Veröffentlichungsnummer: WO 2020/007673

(56) Entgegenhaltungen:
- DE-A1-102014 224 942
- DE-A1-102014 224 943
- DE-A1-102015 210 473
- US-A1- 2004 238 378
- US-A1- 2009 120 161

## Beschreibung

### Stand der Technik

Aus dem Stand der Technik ist eine Vielzahl von Verfahren und Sensoren zum Nachweis mindestens eines Anteils der Messgaskomponente mit gebundenem Sauerstoff in einem Gasgemisch, insbesondere in einem Abgas einer Verbrennungskraftmaschine, durch Erfassen eines Anteils an Sauerstoff, der durch eine Reduktion der Messgaskomponente mit dem gebundenen Sauerstoff erzeugt wird, bekannt.

Sensoren zum Nachweis mindestens eines Anteils der Messgaskomponente mit gebundenem Sauerstoff in einem Gasgemisch, die auch verkürzt oder vereinfacht NOx-Sensoren oder Stickoxid-Sensoren bezeichnet werden, sind beispielsweise in Reif, K., Deitsche, K-H. et al., Kraftfahrtechnisches Taschenbuch, Springer Vieweg, Wiesbaden, 2014, Seite 1338-1347 beschrieben.

Stickoxid-Sensoren (= NOₓ-Sensoren), die heutzutage in der Automobiltechnik eingesetzt werden, funktionieren nach dem Grenzstromprinzip, analog zu Sauerstoff-Sensoren, wie beispielsweise Lambda Sensoren. Ein solcher Stickoxid-Sensor umfasst eine Nernst-Konzentrationszelle, die auch Referenzzelle genannt wird, eine modifizierte Sauerstoffpumpzelle und eine weitere modifizierte Sauerstoffpumpzelle, die sogenannte NOₓ-Zelle. Eine dem Abgas ausgesetzte äußere Pumpelektrode und eine innere Pumpelektrode in einem ersten Hohlraum, der vom Abgas durch eine Diffusionsbarriere getrennt ist, bilden die Sauerstoffpumpzelle. Im ersten Hohlraum befindet sich auch die Nernstelektrode und in einem Referenzgasraum die Referenzelektrode, die zusammen die Nernstzelle bzw. Referenzzelle bilden. Die NOₓ-Zelle umfasst eine NOₓ-Pumpelektrode und eine Gegenelektrode. Die NOₓ-Pumpelektrode befindet sich in einem zweiten Hohlraum, der mit dem ersten inneren Hohlraum verbunden und von diesem durch eine Diffusionsbarriere getrennt ist. Die Gegenelektrode befindet sich in dem Referenzgasraum. Alle Elektroden in dem ersten und zweiten Hohlraum haben einen gemeinsamen Rückleiter.

Bei Betrieb des Stickoxid-Sensor wird der sogenannten O2-Zelle der Sauerstoff aus dem ersten Hohlraum, der über eine Diffusionsbarriere mit dem Abgas verbunden ist, entfernt. Der dadurch resultierende Pumpstrom ist dann proportional zum Sauerstoffgehalt der Umgebungsluft im Messgas- bzw. Abgasstrom. In der NOₓ-Zelle werden die Stickoxide abgepumpt. Das Stickoxid NOₓ, in der in den zweiten Hohlraum befindlichen Atmosphäre, wird durch Anlegen einer konstanten Pumpspannung reduziert bzw. abgebaut. Der durch Reduktion oder Abbau der Messgaskomponente in dem zweiten Hohlraum erzeugte Sauerstoff, der vorzugsweise aus der Reduktion des Stickoxids NOₓ stammt, wird in einen Referenzgasraum abgepumpt. So hat die angelegte Pumpspannung gegen den Widerstand der NOₓ-Zelle und der Konzentration des Stickoxids NOₓ bzw. Sauerstoffs einen Pumpstrom zur Folge, der proportional zum Gehalt an Stickoxid NOₓ bzw. Sauerstoff ist und das NOₓ-Messsignal darstellt.

Der dabei resultierende Pumpstrom I_{P2} ist somit ein Maß für die NOₓ-Konzentration der Umgebungsluft im Messgas- bzw. Abgasstrom. Bei dieser Anordnung ist es wichtig, dass an der Sauerstoffzelle nicht auch die Stickoxide abgepumpt werden, da sonst an der NOₓ-Zelle kein Signal mehr gemessen werden könnte. Dies wird durch eine Gold-Dotierung der O2-Zelle erreicht. Zusätzlich darf die O2-Zelle nur bei niedrigen Pumpspannungen betrieben werden, da sonst wieder NOₓ-Moleküle dissoziiert würden.

Trotz der Vorteile der aus dem Stand der Technik bekannten Sensoren und Verfahren zum Betreiben derselben, beinhalten diese noch Verbesserungspotenzial. Die Temperatur des Sensorelements wird durch eine Pulsweitenmodulation (PWM) der Heizerversorgung (Spannung, Strom) gesteuert. Die Spannung des PWMs wird über einen Feldeffekttransistor (FET) direkt von der Versorgungspannung (12V) der Sensorsteuereinheit (SCU) abgegriffen. Dadurch liegt in der An-Phase des PWMs die SCU-Versorgungsspannung des Systems an den Heizmäander des Sensorelements in der Sensor-Probe an. Der Strom an NOx-Messsignal ist sehr klein wie beispielsweise. 4,5 µA bei 1500 ppm NOx und damit auch äußerst empfindlich gegenüber Störungen und Einkopplungen. Aufgrund der baulichen Nähe der Heizmäander zu der NOx Zelle wird während der An-Phase des PWM-Signals durch kapazitive Kopplung und Leckströme ein Strom auf die IP2 Leitung/Zelle eingeprägt. Durch diese Störung wird ein Offset zu dem tatsächlichen NOx Wert messbar. Die Anforderungen der Umweltbehörden verlangen eine kontinuierliche und verlässliche Diagnose von Leitungsunterbrechungen der IP2 Leitung.

Derartige Sensoren und Verfahren sind beispielsweise aus der DE 10 2015 210 473 A1, der US 2009/120 161 A1 und der US 2004/238 378 A1 bekannt.

### Offenbarung der Erfindung

Es wird daher ein Verfahren zum Betreiben eines Sensors zum Nachweis mindestens eines Anteils einer Messgaskomponente mit gebundenem Sauerstoff in einem Messgas vorgeschlagen, welches die Nachteile bekannter Verfahren zum Betreiben dieser Sensoren zumindest weitgehend vermeidet und das in Intervallen von mindestens 500 ms eine sichere und kontinuierliche Diagnose erlaubt, ohne die NOx-Messwerte zu beeinflussen bzw. zu stören.

Bei einem erfindungsgemäßen Verfahren zum Betreiben eines Sensors zum Nachweis mindestens eines Anteils einer Messgaskomponente mit gebundenem Sauerstoff in einem Messgas, insbesondere in einem Abgas einer Verbrennungskraftmaschine, wobei der Sensor ein Sensorelement umfasst, wobei das Sensorelement eine erste Pumpzelle, die eine äußere Pumpelektrode und eine innere Pumpelektrode aufweist und die an einem ersten Hohlraum anliegt, welcher mit dem Messgas in Verbindung steht, eine Referenzzelle, welche eine Nernst-Elektrode und eine Referenzelektrode aufweist und die an einem Referenzgasraum anliegt, und eine zweite Pumpzelle, die eine Pumpelektrode und eine Gegenelektrode aufweist und die an einem zweiten Hohlraum anliegt, wird ein elektronisches Steuergerät, das zumindest über einen ersten gesonderten Anschluss für die erste Pumpzelle und einen zweiten gesonderten Anschluss für die zweite Pumpzelle verfügt, mit dem Sensorelement verbunden, wobei die erste Pumpzelle mittels einer elektrisch leitenden Verbindung mit dem ersten gesonderten Anschluss verbunden wird, wobei die zweite Pumpzelle mittels einer elektrisch leitenden Verbindung mit dem zweiten gesonderten Anschluss verbunden wird, wobei in der elektrisch leitenden Verbindung, die die zweite Pumpzelle mit dem zweiten gesonderten Anschluss verbindet, ein Messwiderstand vorgesehen wird, wobei mittels des Steuergeräts eine Strom- und/oder Spannungsanregung der zweiten Pumpzelle zum Erzeugen eines Messsignals an dem Messwiderstand durchgeführt wird.

Durch die Strom- und/oder Spannungsanregung der zweiten Pumpzelle wird an dem Messwiderstand ein auswertbares Messsignal erzeugt, das die Unterscheidung zwischen einem offenen oder geschlossenen Stromkreis erlaubt. Mit anderen Worten wird ein IP2-Signal durch eine äußere Anregung erzeugt. Damit lässt sich ein offener Stromkreis an der IP2 Leitung der NOx-Zelle auch während des Messbetriebs sicher erkennen. Auch in Betriebszuständen, in denen der Strom IP2 (nahezu) gleich null ist, wie beispielsweise bei 0 ppm NOx, kann eine offene IP2-Leitung detektiert werden.

Gemäß der ersten Alternative der Erfindung wird an die zweite Pumpzelle eine vorbestimmte elektrische Spannung angelegt, wobei eine Spannungsanregung der zweiten Pumpzelle durchgeführt wird, wobei für eine erste vorbestimmte Zeit die vorbestimmte elektrische Spannung erhöht wird und für eine zweite vorbestimmte Zeit die vorbestimmte elektrische Spannung erniedrigt wird, wobei ein Integral der angelegten elektrischen Spannung für die erste vorbestimmte Zeit und die zweite vorbestimmte Zeit einen Wert von Null aufweist. Im Messzustand des NOx-Sensors wird somit von der Hardware die Spannung U_{P2} an der NOx-Zelle für kurze Zeit um ein bestimmtes Potential erhöht und danach für die gleiche Zeit in die entgegengesetzte Potentialrichtung minimiert. Es spielt keine Rolle ob der positive Spannungspuls oder der negative Spannungspuls zuerst durchgeführt wird. Durch Anregung der Spannung gegenüber dem normalen Potential kommt es bei geschlossener Leitung mit einem Gasgemisch, auch bei 0% O2, H2O ≥1% und NOx = 0 ppm, zu einem messbaren Stromfluss. Ist die IP2-Leitung aufgetrennt, kann kein Strom mehr fließen, auch nicht bei einer zeitlichen Anregung durch einen Puls. Dies gilt als Hinweis für einen offenen Stromkreis.

Gemäß der ersten Alternative der Erfindung wird die elektrisch leitende Verbindung, die die zweite Pumpzelle mit dem zweiten gesonderten Anschluss verbindet, als intakt identifiziert wird, falls das Messsignal für die erste vorbestimmte Zeit und die zweite vorbestimmte Zeit eine Wert von ungleich Null aufweist, und als defekt identifiziert wird, falls das Messsignal für die erste vorbestimmte Zeit und die zweite vorbestimmte Zeit eine Wert von Null aufweist.

Das Integral der Spannungsamplitude über beide Pulse muss über die Zeit beider Pulse null ergeben, also gleichanteilsfrei sein, um einen geschlossenen Stromkreis anzuzeigen. Andernfalls liegt ein Hinweis auf einen offenen Stromkreis vor.

Gemäß einer zweiten Alternative der Erfindung wird in die zweite Pumpzelle ein vorbestimmter elektrischer Strom eingeprägt, wobei eine Stromanregung der zweiten Pumpzelle durchgeführt wird, wobei für eine erste vorbestimmte Zeit der vorbestimmte elektrische Strom erhöht wird und für eine zweite vorbestimmte Zeit der vorbestimmte elektrische Strom erniedrigt wird, wobei die erste vorbestimmte Zeit und die zweite vorbestimmte Zeit identisch lang sind. Eine Alternative zum Spannungspuls ist ein Strompuls an der IP2 Leitung. Im Messzustand des NOx-Sensors wird von der Hardware ein Strompuls für kurze Zeit um einen bestimmten Wert erhöht und danach für die gleiche Zeit in die entgegengesetzte Richtung minimiert. Es spielt keine Rolle ob der positive Strompuls oder der negative Strompuls zuerst durchgeführt wird. Durch das Einprägen eines Strompulses (Pumpstrom) wird ein Spannungshub an der IP2-Leitung messbar sein. Dieser verhält sich bei geschlossenem Stromkreis anders als bei einem offenen Stromkreis. Dies kann als Unterscheidungsmerkmal für die Detektion eines offenen Stromkreises an der IP2 -Leitung hergenommen werden.

Dabei wird die zweite elektrisch leitende Verbindung als intakt identifiziert, falls eine an die zweite Pumpzelle angelegte elektrische Spannung für die erste vorbestimmte Zeit und für die zweite vorbestimmte Zeit einen Schwellwert unterschreitet, und als defekt identifiziert wird, falls eine an die zweite Pumpzelle angelegte elektrische Spannung für die erste vorbestimmte Zeit und für die zweite vorbestimmte Zeit einen Schwellwert überschreitet. Das Integral des Stromes über beide Pulse muss über die Zeit beider Pulse null ergeben, um keine Ungleichgewicht in der NOx Zelle hervorzurufen, durch einseitiges aufpumpen bzw. abpumpen. Durch das Einprägen eines Strompulses (Pumpstrom) wird ein Spannungshub an der IP2-Leitung messbar sein. Dieser verhält sich bei geschlossenem Stromkreis anders als bei einem offenen Stromkreis. So fällt der Spannungshub bei geschlossenem Stromkreis niedriger aus als bei einem offenen Stromkreis. Dies kann als Unterscheidungsmerkmal für die Detektion eines offenen Stromkreises an der IP2 -Leitung hergenommen werden.

Gemäß einer dritten Alternative der Erfindung wird an die zweite Pumpzelle eine vorbestimmte elektrische Spannung angelegt, wobei eine Spannungsanregung der zweiten Pumpzelle durchgeführt wird, wobei die Spannungsanregung eine periodische Veränderung der vorbestimmten elektrischen Spannung umfasst. Das Anlegen einer Spannungsanregung auf der IP2 Leitung verursacht eine deutliche Stromänderung an der NOx-Zelle. Dabei kann die Amplitude des modulierten Signals sehr klein sein, wenn die Frequenz groß genug ist. Die Frequenz auf der IP2-Leitung muss nicht zwingend über den gesamten Zeitraum angeregt werden. Das Anlegung der höherfrequenten Spannungsänderung im Bereich um nahezu 0 ppm NOx reicht für die elektrische Diagnose aus. Wird auf dem Rohsignal, besonders bei 0% O2, H2O ≥1% und NOx = 0 ppm, ein durch die Frequenz angeregter Strom messbar, so ist die IP2-Leitung in Ordnung. Ist auf dem Rohsignal kein Strom messbar, so muss an der NOx-Zelle Leitung ein offener Stromkreis anliegen. Die Amplitudenform ist dann unwesentlich. Jedoch, je größer die zeitliche Änderung d/dt U(t) der Spannung, umso stärker ist die Stromänderung.

Bei einer Weiterbildung ist die Periodendauer kleiner als eine elektrochemische Zeitkonstante der zweiten Pumpzelle. Das Anlegen einer Spannungsanregung auf der IP2-Leitung, deren Periodendauer weit unter der (elektrochemischen) Zeitkonstante der NOx-Zelle liegt, verursacht eine deutliche Stromänderung an der NOx-Zelle. Dabei kann die Amplitude des modulierten Signals sehr klein sein, wenn die Frequenz groß genug ist. Die Frequenz der Spannungsänderung sollte so gewählt werden, dass das NOx-Ausgangssignal nicht gestört wird. Die Frequenz auf der IP2-Leitung muss nicht zwingend über den gesamten Zeitraum angeregt werden. Das Anlegung der höherfrequenten Spannungsänderung im Bereich um nahezu 0 ppm NOx reicht für die elektrische Diagnose aus. Wird auf dem Rohsignal, besonders bei 0% O2, H2O ≥1% und NOx = 0 ppm, ein durch die Frequenz angeregter Strom messbar, so ist die IP2-Leitung in Ordnung. Ist auf dem Rohsignal kein Strom messbar, so muss an der NOx-Zelle Leitung ein offener Stromkreis anliegen. Die Amplitudenform ist dann unwesentlich. Jedoch, je größer die zeitliche Änderung d/dt U(t) der Spannung, umso stärker ist die Stromänderung.

Bei einer Weiterbildung wird das Messsignal mittels eines Tiefpassfilters gefiltert. Typischerweise wird das Rohsignal mit einem Tiefpass gefiltert und über die Schnittstelle des Sensorsteuergerätes an das Motorsteuergerät übertragen.

Durch die Frequenzeinkopplung wird die Änderung des Stromes auf dem Rohsignal des IP2-Stroms sichtbar und kann für die elektrische Diagnose verwendet werden. Die NOx-Werte, die sich aus dem IP2-Strom ergeben werden, bevor sie auf dem CAN gesendet werden, mit einem Tiefpassfilter gefiltert. Auf dem gefilterten Signal ist diese höherfrequente Stromänderung dann nicht mehr sichtbar.

Bei einer Weiterbildung wird die vorbestimmte elektrische Spannung mit einer Frequenz verändert, die größer als die Bandbreite des Tiefpassfilters ist. Sinnvollerweise sollte die Anregungsfrequenz aber auch innerhalb der Bandbreite des Analog-Digital-Wandlers (ADC - analog-to-digital converter) liegen. Durch die Frequenzeinkopplung wird die Änderung des Stromes auf dem Rohsignal des IP2-Stroms sichtbar und kann für die elektrische Diagnose verwendet werden. Die NOx-Werte, die sich aus dem IP2-Strom ergeben werden, bevor sie auf dem CAN gesendet werden, werden mit einem Tiefpassfilter gefiltert. Auf dem gefilterten Signal ist diese höherfrequente Stromänderung dann nicht mehr sichtbar.

Gemäß der driten Alternative der Erfindung wird die elektrisch leitende Verbindung, die die zweite Pumpzelle mit dem zweiten gesonderten Anschluss verbindet, als intakt identifiziert, falls das (ungefilterte) Messsignal eine periodische Veränderung zeigt, und wird als defekt identifiziert, falls das Messsignal keine periodische Veränderung zeigt. Wird auf dem Rohsignal, besonders bei 0% O2, H2O ≥1% und NOx = 0 ppm, ein durch die Frequenz angeregter Strom messbar, so ist die IP2-Leitung in Ordnung. Ist auf dem Rohsignal kein Strom messbar, so muss an der IP2-Leitung ein offener Stromkreis anliegen. Die Amplitudenform ist dann unwesentlich. Jedoch, je größer die zeitliche Änderung d/dt U(t) der Spannung, umso stärker ist die Stromänderung.

Unter einem Festelektrolyten ist im Rahmen der vorliegenden Erfindung ein Körper oder Gegenstand mit elektrolytischen Eigenschaften, also mit Ionen leitenden Eigenschaften, zu verstehen. Insbesondere kann es sich um einen keramischen Festelektrolyten handeln. Dies umfasst auch das Rohmaterial eines Festelektrolyten und daher die Ausbildung als so genannter Grünling oder Braunling, der erst nach einem Sintern zu einem Festelektrolyten wird.

Insbesondere kann der Festelektrolyt als Festelektrolytschicht oder aus mehreren Festelektrolytschichten ausgebildet sei. Unter einer Schicht ist im Rahmen der vorliegenden Erfindung eine einheitliche Masse in flächenhafter Ausdehnung einer gewissen Höhe zu verstehen, die über, unter oder zwischen anderen Elementen liegt.

Unter einer Elektrode ist im Rahmen der vorliegenden Erfindung allgemein ein Element zu verstehen, welches in der Lage ist, den Festelektrolyten derart zu kontaktieren, dass durch den Festelektrolyten und die Elektrode ein Strom aufrechterhalten werden kann. Dementsprechend kann die Elektrode ein Element umfassen, an welchem die Ionen in den Festelektrolyten eingebaut und/oder aus dem Festelektrolyten ausgebaut werden können. Typischerweise umfassen die Elektroden eine Edelmetallelektrode, welche beispielsweise als Metall-Keramik-Elektrode auf dem Festelektrolyten aufgebracht sein kann oder auf andere Weise mit dem Festelektrolyten in Verbindung stehen kann. Typische Elektrodenmaterialien sind Platin-Cermet-Elektroden. Auch andere Edelmetalle, wie beispielsweise Gold oder Palladium, sind jedoch grundsätzlich einsetzbar.

Unter einem Heizelement ist im Rahmen der vorliegenden Erfindung ein Element zu verstehen, das zum Erwärmen des Festelektrolyten und der Elektroden auf mindestens ihre Funktionstemperatur und vorzugsweise auf ihre Betriebstemperatur dient. Die Funktionstemperatur ist diejenige Temperatur, ab der der Festelektrolyt für Ionen leitend wird und die ungefähr 350 °C beträgt. Davon ist die Betriebstemperatur zu unterscheiden, die diejenige Temperatur ist, bei der das Sensorelement üblicherweise betrieben wird und die höher ist als die Funktionstemperatur. Die Betriebstemperatur kann beispielsweise von 700 °C bis 950 °C sein. Das Heizelement kann einen Heizbereich und mindestens eine Zuleitungsbahn umfassen. Unter einem Heizbereich ist im Rahmen der vorliegenden Erfindung der Bereich des Heizelements zu verstehen, der in dem Schichtaufbau entlang einer zu der Oberfläche des Sensorelements senkrechten Richtung mit einer Elektrode überlappt. Üblicherweise erwärmt sich der Heizbereich während des Betriebs stärker als die Zuleitungsbahn, so dass diese unterscheidbar sind. Die unterschiedliche Erwärmung kann beispielsweise dadurch realisiert werden, dass der Heizbereich einen höheren elektrischen Widerstand aufweist als die Zuleitungsbahn. Der Heizbereich und/oder die Zuleitung sind beispielsweise als elektrische Widerstandsbahn ausgebildet und erwärmen sich durch Anlegen einer elektrischen Spannung. Das Heizelement kann beispielsweise aus einem Platin-Cermet hergestellt sein. Die Erfindung ist direkt durch eine verkürzte Wartezeit bis zum Erreichen der Betriebsbereitschaft nach Start des Sensors nachweisbar. Die jeweiligen Potenziale können an den Zuleitungen gemessen werden.

Unter einer Spannungsanregung der zweiten Pumpzelle ist im Rahmen der vorliegenden Erfindung das Anlegen einer elektrischen Spannung ungleich 0 V an die zweite Pumpzelle bzw. die elektrische Leitung zur NOx-Gegenelektrode zu verstehen. Die Spannungsanregung erzeugt einen Stromfluss durch die Pumpzelle und die elektrische Leitung zur NOx-Gegenelektrode. Dieser Stromfluss bewirkt an einem Messwiderstand in dieser Leitung ein erfassbares Messsignal.

Unter einer Stromanregung der zweiten Pumpzelle ist im Rahmen der vorliegenden Erfindung das Einprägen eines elektrischen Stroms ungleich 0 A in die zweite Pumpzelle bzw. die elektrische Leitung zur NOx-Gegenelektrode zu verstehen. Durch das Einprägen eines Strompulses (Pumpstrom) wird ein Spannungshub an der elektrischen Leitung zur NOx-Gegenelektrode bewirkt, der an einem Messwiderstand in dieser Leitung messbar ist. Dieser verhält sich bei geschlossenem Stromkreis anders als bei einem offenen Stromkreis.

Unter einem Tiefpass ist im Rahmen der vorliegenden Erfindung ein Filter zu verstehen, der niederfrequente Signalanteile unterhalb seiner Grenzfrequenz durchlässt, während hochfrequente Signalanteile gedämpft werden. Aufgabe des Tiefpassfilters ist, den Verlauf des Signals des Differenzsignals über das Zeitintervall der Störung zu summieren. Ist das Differenzsignal klein, wird das Kompensationsstromsignal gegen Null gehen. Eine einfache Tierfpass-Umsetzung kann zum Beispiel mit einem Leaky-Integrator erreicht werden. In einer alternativen Umsetzung können die Eigenschaften des Tiefpasses in Abhängigkeit von der Größe des Eingangssignals geändert werden.

Die Erfindung ist gut und einfach durch die Überwachung der elektrischen Signale auf der NOx-Zellen-Leitung nachweisbar. Werden während des normalen Messbetriebs spezielle ein Spannungspuls/Strompuls auf der Leitung zwischen Sensor und Steuergerät mit einem Oszilloskop gemessen, so werden die in dieser Erfindung beschriebenen Schaltungen und Verfahren verwendet.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind.

Es zeigen:
Figur 1 einen prinzipiellen Aufbau eines erfindungsgemäßen Sensors,

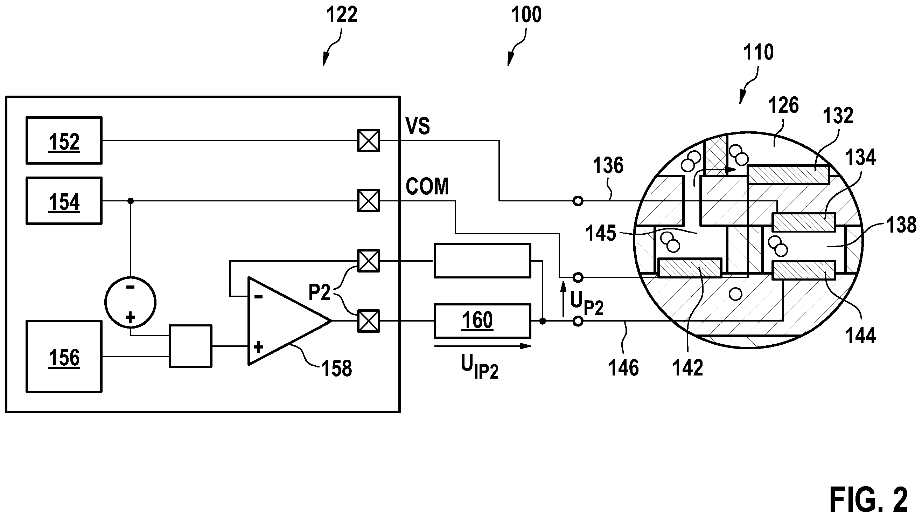

Figur 2 einen Teil des Sensors mit einem Teil eines daran angeschlossenen Steuergeräts,
Figur 3 ein erstes Beispiel für einen zeitlichen Verlauf von elektrischen Spannungen und Messsignal bei dem Sensor,
Figur 4 ein zweites Beispiel für einen zeitlichen Verlauf von elektrischen Spannungen und Messsignal bei dem Sensor und
Figur 5 ein drittes Beispiel für einen zeitlichen Verlauf von elektrischen Spannungen, elektrischen Strömen und Messsignal bei dem Sensor.

### Ausführungsformen der Erfindung

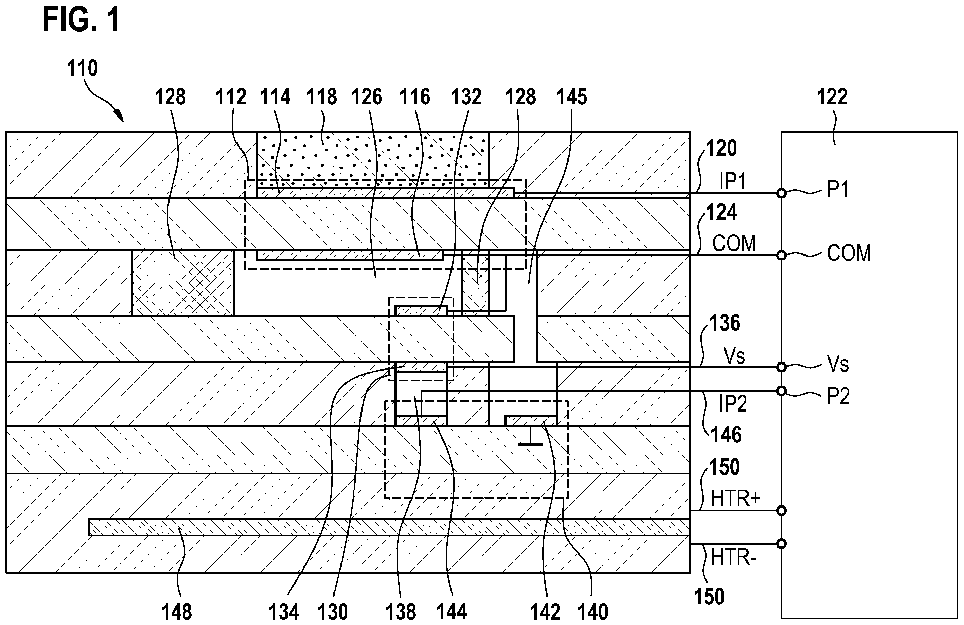

Figur 1 zeigt einen prinzipiellen Aufbau eines erfindungsgemäßen Sensors 100, welcher zur Durchführung des erfindungsgemäßen Verfahrens besonders geeignet ist.

Der Sensor 100, welcher zum Nachweis mindestens eines Anteils einer Messgaskomponente mit gebundenem Sauerstoff, im Folgenden beispielhaft als Stickoxid NOx bezeichnet, in einem Gasgemisch, beispielhaft einem Abgas einer Verbrennungskraftmaschine, eingerichtet ist, umfasst hierzu ein Sensorelement 110 eine erste Pumpzelle 112, welche zwischen einer äußeren Pumpelektrode 114 und einer inneren Pumpelektrode 116 ausgebildet ist. Die äußere Pumpelektrode 114, welche mittels einer porösen Aluminiumoxidschicht 118 von der Umgebung des Sensors 100 getrennt ist, verfügt hierbei über eine erste elektrisch leitende Verbindung 120, über welche sich ein erster Pumpstrom I_{P1} in der ersten Pumpzelle 112 erzeugen lässt. Die erste elektrisch leitende Verbindung 120 ist hierzu mit einem ersten Anschluss P1 eines externen elektronischen Steuergeräts 122 verbunden. Um einen vollständigen Stromkreis zu erhalten, verfügt die innere Pumpelektrode 116 ebenfalls über eine zweite elektrisch leitende Verbindung 124, welche zu einem gemeinsamen Anschluss COM des externen elektronischen Steuergeräts 122 führt. Die erste Pumpzelle 112 liegt an einem ersten Hohlraum 126 an, der sich im Inneren des Sensorelements 110 befindet und mit dem Messgas in Verbindung steht. Durch Erzeugen des ersten Pumpstroms I_{P1} in der ersten Pumpzelle 112 lässt sich ein erster Anteil von Sauerstoffionen, welche aus molekularem Sauerstoff aus dem Gasgemisch gebildet werden, zwischen dem ersten Hohlraum 126 und der Umgebung des Sensors 100 transportieren. In dem Eintrittsweg aus der Umgebung zu dem ersten Hohlraum 126 sind zwei Diffusionsbarrieren 128 vorhanden.

Das Sensorelement 110 weist weiterhin eine elektrische Referenzzelle 130 auf, welche eine Nernst-Elektrode 132 und eine Referenzelektrode 134 aufweist. Während die Nernst-Elektrode 132 über die zweite elektrisch leitende Verbindung 124 zusammen mit der inneren Pumpelektrode 116 zu dem gemeinsamen Anschluss COM verfügt, weist die Referenzelektrode 134 eine gesonderte dritte elektrisch leitende Verbindung 136 zu einer Versorgungsspannung Vs auf, welche über einen Anschluss Vs des externen elektronischen Steuergeräts 122 die erforderliche Versorgungsspannung Vs bereitstellt. Die Referenzzelle 130 liegt an einem Referenzgasraum 138 an. Ein zweiter Anteil der Sauerstoffionen aus dem ersten Hohlraum 126 und/oder aus der Umgebung des Sensors 100 wird in den Referenzgasraum 138 durch Anlegen eines Referenz-Pumpstroms zwischen dem Anschluss Vs und dem gemeinsamen Anschluss COM transportiert. Hierbei wird der Wert für den Referenz-Pumpstrom derart eingestellt, dass sich ein festgelegter Anteil der Sauerstoffionen in dem Referenzgasraum 138 ausbildet. Vorzugsweise wird in diesem Zusammenhang auch der Wert für den ersten Pumpstrom I_{P1} derart eingestellt, dass sich ein festgelegtes Verhältnis zwischen dem ersten Anteil der Sauerstoffionen in dem ersten Hohlraum 126 und dem zweiten Anteil der Sauerstoffionen in dem Referenzgasraum 138 ergibt.

Die in dem Gasgemisch weiterhin enthaltene Messgaskomponente Stickoxid NOₓ mit dem gebundenen Sauerstoff gelangt, insbesondere durch Diffusion, weitgehend unbeeinflusst in eine zweite Pumpzelle 140 des Sensorelements 110, welche auch als "NOₓ-Pumpzelle" bezeichnet werden kann. Die zweite Pumpzelle 140 weist eine NOₓ-Pumpelektrode 142 und eine NOₓ-Gegenelektrode 144 auf und liegt an einem zweiten Hohlraum 145 im Inneren des Sensorelements 110 an. Der zweite Hohlraum 145 ist von dem ersten Hohlraum 126 durch eine der Diffusionsbarrieren 128 getrennt. Wenigstens eine der beiden Elektroden NOₓ-Pumpelektrode 142 und/oder NOₓ-Gegenelektrode 144 sind derart ausgestaltet, dass bei Anlegen einer Spannung mittels Katalyse aus der Messgaskomponente NOₓ weiterer molekularer Sauerstoff erzeugt werden kann, welcher in der zweiten Pumpzelle 140 gebildet wird.

Während die NOₓ-Pumpelektrode 142 eine elektrisch leitende Verbindung aufweist, welche zu dem gemeinsamen Anschluss COM führt, weist die NOₓ-Gegenelektrode 144 eine vierte elektrisch leitende Verbindung 146 auf, über welche ein zweiter Pumpstrom I_{P2} an die zweite Pumpzelle 140 angelegt werden kann. Die vierte elektrisch leitende Verbindung 146 ist hierzu mit einem zweiten Anschluss P2 des externen elektronischen Steuergeräts 122 verbunden. Bei Anlegen eines zweiten Pumpstroms I_{P2} an die zweite Pumpzelle 140 wird ein Anteil von weiteren Sauerstoffionen, welche aus dem weiteren molekularen Sauerstoff gebildet wurden, in den Referenzgasraum 138 transportiert.

Das Sensorelement 110 verfügt weiterhin über ein Heizelement 148, welches eine Heizleitung 150 mit den Leitungen HTR+ und HTR- aufweist, über welche ein Heizstrom in das Heizelement 148 eingebracht werden kann, welches mittels Erzeugen einer Heizleistung das Sensorelement 110 auf die gewünschte Temperatur bringen kann.

Das Steuergerät 122 weist einen Analog-Digital-Wandler 152 auf, der mit dem Anschluss Vs für die Versorgungsspannung verbunden ist. Das Steuergerät 122 weist weiterhin eine COM-Spannungsquelle 154 auf, die mit dem gemeinsamen Anschluss COM verbunden ist. Das Steuergerät 122 weist weiterhin eine Anregungssignalquelle 156 auf, die mit dem Pluspol eines Operationsverstärkers 158 verbunden ist. Bei dem gezeigten Ausführungsbeispiel ist der Operationsverstärker 158 ein Spannungsfolger. Die COM-Spannungsquelle 154 ist ebenfalls mit dem Pluspol des Operationsverstärkers 158 verbunden. Der Operationsverstärker 158 ist wiederum mit dem zweiten Anschluss P2 verbunden. In der vierten elektrischen Leitung 146 ist zwischen dem zweiten Anschluss P2 und der Gegenelektrode 144 ein Messwiderstand 160 angeordnet.

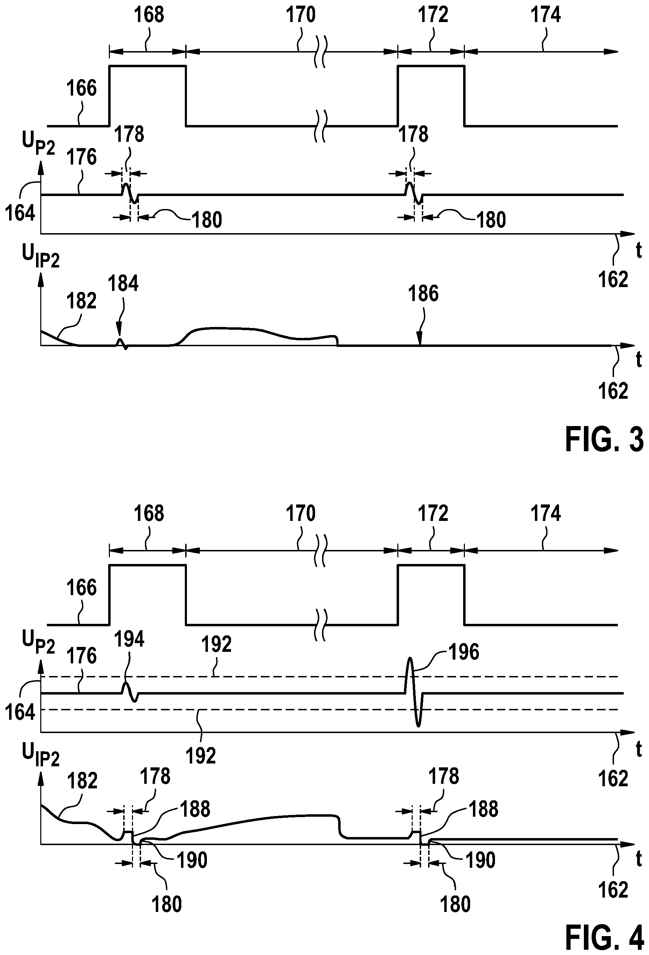

Figur 3 zeigt ein erstes Beispiel für einen zeitlichen Verlauf von elektrischen Spannungen und Messsignal bei dem Sensor 100. Auf der X-Achse 162 ist die Zeit aufgetragen. Auf der Y-Achse 164 sind von oben nach unten gesehen die Heizspannung des Heizelements 148, die an die zweite Pumpzelle 140 angelegte elektrische Spannung U_{P2} und der Spannungsabfall U_{IP2} am Messwiderstand 160 aufgetragen. Der Spannungsabfall U_{IP2} am Messwiderstand 160 stellt das Messsignal dar. Die Temperatur des Sensorelements 110 wird durch eine Pulsweitenmodulation (PWM) der Heizerversorgung (Spannung bzw. Strom) gesteuert. Entsprechend zeigt eine Kurve 166, die die Heizspannung des Heizelements 148 darstellt, zumindest eine erste Phase bzw. einen ersten Zeitraum 168, in dem an das Heizelement 148 eine elektrische Spannung angelegt ist und das Heizelement 148 somit angeschaltet ist, und eine zweite Phase bzw. einen zweiten Zeitraum 170, in dem an das Heizelement 148 keine elektrische Spannung angelegt ist und das Heizelement 148 somit ausgeschaltet ist. Dabei folgt der zweite Zeitraum 170 auf den ersten Zeitraum 168. Eine Summe aus erstem Zeitraum 168 und zweitem Zeitraum 170 ist beispielsweise 500 ms. Bei dem gezeigten Ausführungsbeispiel zeigt die Kurve 166 weiterhin eine dritte Phase bzw. einen dritten Zeitraum 172, in dem an das Heizelement 148 eine elektrische Spannung angelegt ist und das Heizelement 148 somit angeschaltet ist, und eine vierte Phase bzw. einen vierten Zeitraum 174, in dem an das Heizelement 148 keine elektrische Spannung angelegt ist und das Heizelement 148 somit ausgeschaltet ist. Dabei folgt der vierte Zeitraum 174 auf den dritten Zeitraum 172. Eine Summe aus drittem Zeitraum 172 und viertem Zeitraum 174 ist beispielsweise 500 ms.

Zur Durchführung einer Diagnose der vierten elektrischen Leitung 146, die den zweiten gesonderten Anschluss P2 mit der Pumpzelle 140 bzw. der Gegenelektrode 144 verbindet, wird mittels des Steuergeräts 122 eine Spannungsanregung der zweiten Pumpzelle 140 zum Erzeugen eines Messsignals an dem Messwiderstand 160 durchgeführt. So wird an die zweite Pumpzelle 140 eine vorbestimmte elektrische Spannung 176 über den gemeinsamen Anschluss COM angelegt. Es wird eine Spannungsanregung der zweiten Pumpzelle 140 mittels der Anregungssignalquelle 156 durchgeführt. Für eine erste vorbestimmte Zeit 178 wird die vorbestimmte elektrische Spannung erhöht und für eine zweite vorbestimmte Zeit 180 wird die vorbestimmte elektrische Spannung 176 erniedrigt. Die exakte Reihenfolge der Veränderung der Spannung ist nicht relevant. So kann alternativ die vorbestimmte elektrische Spannung zuerst erniedrigt bzw. abgesenkt und dann erhöht werden. Die Spannungsanregung wird so durchgeführt, dass ein Integral der angelegten elektrischen Spannung für die erste vorbestimmte Zeit 178 und die zweite vorbestimmte Zeit 180 einen Wert von Null aufweist. Dies kann durch gleiche Spannungsamplituden über identisch lange Zeiträume realisiert werden. Die erste vorbestimmte Zeit 178 und die zweite vorbestimmte Zeit 180 sind bei dem gezeigten Ausführungsbeispiel identisch lang. Beispielsweise wird ausgehend von einer an die zweite Pumpzelle 140 angelegten elektrischen Spannung von 425 mV für die erste vorbestimmte Zeit 178 die elektrische Spannung auf 700 mV erhöht und dann für die zweite vorbestimmte Zeit 180 auf 150 mV abgesenkt. Dies geschieht in einem Zeitraum, in dem das Heizelement 148 angeschaltet ist, beispielsweise in dem ersten Zeitraum 168 und dem dritten Zeitraum 172. Dies erzeugt ein Messsignal 182 an dem Messwiderstand 160. Die vierte elektrisch leitende Verbindung 146 wird als intakt identifiziert, falls das Messsignal 182 für die erste vorbestimmte Zeit 178 und die zweite vorbestimmte Zeit 180 einen Wert von ungleich Null aufweist, und wird als defekt identifiziert, falls das Messsignal 182 für die erste vorbestimmte Zeit 178 und die zweite vorbestimmte Zeit 180 einen Wert von Null aufweist. Bei dem gezeigten Ausführungsbeispiel weist das Messsignal 182 bei der Spannungsanregung in dem ersten Zeitraum 168 einen durch einen Pfeil angedeuteten, annähernd sinusförmigen Verlauf 184 auf und weist in dem dritten Zeitraum 172 einen Wert von Null auf, so dass sich das Signal an de durch einen Pfeil angedeuteten Stelle 186 nicht verändert. Dies bedeutet, dass an der Stelle 186 kein Strom fließt, was auf eine Unterbrechung der vierten elektrischen Leitung 146 hindeutet.

Bei einer Modifikation umfasst die Spannungsanregung eine einzige Veränderung der vorbestimmten elektrischen Spannung für eine vorbestimmte Zeit. So wird die vorbestimmte elektrische Spannung für die vorbestimmte Zeit erhöht. Die vierte elektrisch leitende Verbindung 146 wird dabei als intakt identifiziert wird, falls das Messsignal 182 für die vorbestimmte Zeit eine Wert von ungleich Null aufweist, und wird als defekt identifiziert, falls das Messsignal 182 für die vorbestimmte Zeit einen Wert von Null aufweist. Es wird somit im Messzustand des Sensors 100 von der Hardware die vorbestimmte elektrische Spannung für kurze Zeit um ein bestimmtes Potential erhöht in Form eines einmaligen Pulses in eine Richtung. Dabei kommt es nicht auf die Dauer des Pulses an, sondern auf die Spannungsänderung. Der Vorteil dabei ist, dass die Zeit, in der das Messsignal 182 durch den Spannungshub verfälscht wird, geringer ist als bei einem zusätzlichen Gegenpuls in Form eines gleichanteilsfreien Pulses.

Figur 4 zeigt ein zweites Beispiel für einen zeitlichen Verlauf von elektrischen Spannungen und Messsignal bei dem Sensor 100. Es werden lediglich die Unterschiede zu dem in Figur 3 gezeigten Ausführungsbeispiel beschrieben. Gleiche Bauteile bzw. Merkmale sind mit gleichen Bezugszeichen versehen. Auf der X-Achse 162 ist die Zeit aufgetragen. Auf der Y-Achse 164 sind von oben nach unten gesehen die Heizspannung des Heizelements 148, die an die zweite Pumpzelle 140 angelegte elektrische Spannung U_{P2} und der Spannungsabfall U_{IP2} am Messwiderstand 160 aufgetragen. Der Spannungsabfall U_{IP2} am Messwiderstand 160 stellt das Messsignal dar. Die Temperatur des Sensorelements 110 wird durch eine Pulsweitenmodulation (PWM) der Heizerversorgung (Spannung bzw. Strom) gesteuert. Entsprechend zeigt eine Kurve 166, die die Heizspannung des Heizelements 148 darstellt, zumindest eine erste Phase bzw. einen ersten Zeitraum 168, in dem an das Heizelement 148 eine elektrische Spannung angelegt ist und das Heizelement 148 somit angeschaltet ist, und eine zweite Phase bzw. einen zweiten Zeitraum 170, in dem an das Heizelement 148 keine elektrische Spannung angelegt ist und das Heizelement 148 somit ausgeschaltet ist. Dabei folgt der zweite Zeitraum 170 auf den ersten Zeitraum 168. Eine Summe aus erstem Zeitraum 168 und zweitem Zeitraum 170 ist beispielsweise 500 ms. Bei dem gezeigten Ausführungsbeispiel zeigt die Kurve 166 weiterhin eine dritte Phase bzw. einen dritten Zeitraum 172, in dem an das Heizelement 148 eine elektrische Spannung angelegt ist und das Heizelement 148 somit angeschaltet ist, und eine vierte Phase bzw. einen vierten Zeitraum 174, in dem an das Heizelement 148 keine elektrische Spannung angelegt ist und das Heizelement 148 somit ausgeschaltet ist. Dabei folgt der vierte Zeitraum 174 auf den dritten Zeitraum 172. Eine Summe aus drittem Zeitraum 172 und viertem Zeitraum 174 ist beispielsweise 500 ms.

Zur Durchführung einer Diagnose der vierten elektrischen Leitung 146, die den zweiten gesonderten Anschluss P2 mit der Pumpzelle 140 bzw. der Gegenelektrode 144 verbindet, wird mittels des Steuergeräts 122 eine Stromanregung der zweiten Pumpzelle 140 zum Erzeugen eines Messsignals an dem Messwiderstand 160 durchgeführt. So wird in die zweite Pumpzelle 140 ein vorbestimmter elektrischer Strom über den gemeinsamen Anschluss COM angelegt. Es wird eine Stromanregung der zweiten Pumpzelle 140 mittels der Anregungssignalquelle 156 durchgeführt. Für eine erste vorbestimmte Zeit 178 wird der vorbestimmte elektrische Strom erhöht und für eine zweite vorbestimmte Zeit 180 wird der vorbestimmte elektrische Strom erniedrigt. Die exakte Reihenfolge der Veränderung des Stroms ist nicht relevant. So kann alternativ der vorbestimmte elektrische Strom zuerst erniedrigt bzw. abgesenkt und dann erhöht werden. Die Stromanregung wird so durchgeführt, dass ein Integral des eingeprägten elektrischen Stroms für die erste vorbestimmte Zeit 178 und die zweite vorbestimmte Zeit 180 einen Wert von Null aufweist. Dies kann durch gleiche Stromamplituden über identisch lange Zeiträume realisiert werden. Die erste vorbestimmte Zeit 178 und die zweite vorbestimmte Zeit 180 sind bei dem gezeigten Ausführungsbeispiel identisch lang. Beispielsweise wird ausgehend von einem in die zweite Pumpzelle 140 eingeprägten elektrischen Strom für die erste vorbestimmte Zeit 178 der elektrische Strom erhöht und dann für die zweite vorbestimmte Zeit 180 abgesenkt. Dies geschieht in einem Zeitraum, in dem das Heizelement 148 angeschaltet ist, beispielsweise in dem ersten Zeitraum 168 und dem dritten Zeitraum 172. Die Stromanregung ist an einem positiven Peak 188 und einem nachfolgenden negativen Peak 190 des Messsignals 182 an dem Messwiderstand 160 erkennbar. Die Stromanregung erzeugt einen Spannungshub in der an die zweite Pumpzelle angelegten Spannung U_{P2}. Die vierte elektrisch leitende Verbindung 146 wird als intakt identifiziert, falls die an die zweite Pumpzelle angelegten Spannung U_{P2} in der ersten vorbestimmte Zeit 178 und der zweiten vorbestimmten Zeit 180 einen Schwellwert 192 unterschreitet und wird als defekt identifiziert, falls die an die zweite Pumpzelle angelegten Spannung U_{P2} in der ersten vorbestimmte Zeit 178 und der zweiten vorbestimmten Zeit 180 einen Schwellwert 192 überschreitet. Der Schwellwert 192 ist dabei als Größe der Amplitude bzw. dem Betrag der Spannungsänderung definiert. Bei dem gezeigten Ausführungsbeispiel weist die an die zweite Pumpzelle angelegten Spannung U_{P2} bei der Stromanregung in dem ersten Zeitraum 168 einen durch einen Pfeil angedeuteten, annähernd sinusförmigen Spannungshub 194 auf, der den Schwellwert 192 unterschreitet, und weist in dem dritten Zeitraum 172 einen durch einen Pfeil angedeuteten, annähernd sinusförmigen Spannungshub 196 auf, der den Schwellwert 192 überschreitet.

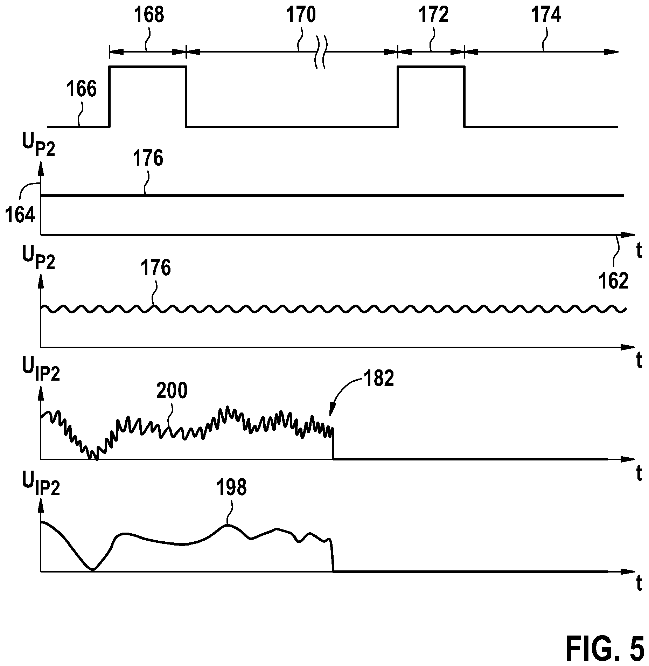

Figur 5 zeigt ein drittes Beispiel für einen zeitlichen Verlauf von elektrischen Spannungen und Messsignal bei dem Sensor 100. Es werden lediglich die Unterschiede zu dem in Figur 3 gezeigten Ausführungsbeispiel beschrieben. Gleiche Bauteile bzw. Merkmale sind mit gleichen Bezugszeichen versehen. Auf der X-Achse 162 ist die Zeit aufgetragen. Auf der Y-Achse 164 sind von oben nach unten gesehen die Heizspannung des Heizelements 148, die an die zweite Pumpzelle 140 angelegte elektrische Spannung U_{P2} ohne Spannungsanregung, die an die zweite Pumpzelle 140 angelegte elektrische Spannung U_{P2} mit Spannungsanregung, der Spannungsabfall U_{IP2} am Messwiderstand 160 und der Spannungsabfall U_{IP2} am Messwiderstand 160 nach Tiefpassfilterung aufgetragen. Der Spannungsabfall U_{IP2} am Messwiderstand 160 stellt das Messsignal dar. Die Temperatur des Sensorelements 110 wird durch eine Pulsweitenmodulation (PWM) der Heizerversorgung (Spannung bzw. Strom) gesteuert. Entsprechend zeigt eine Kurve 166, die die Heizspannung des Heizelements 148 darstellt, zumindest eine erste Phase bzw. einen ersten Zeitraum 168, in dem an das Heizelement 148 eine elektrische Spannung angelegt ist und das Heizelement 148 somit angeschaltet ist, und eine zweite Phase bzw. einen zweiten Zeitraum 170, in dem an das Heizelement 148 keine elektrische Spannung angelegt ist und das Heizelement 148 somit ausgeschaltet ist. Dabei folgt der zweite Zeitraum 170 auf den ersten Zeitraum 168. Eine Summe aus erstem Zeitraum 168 und zweitem Zeitraum 170 ist beispielsweise 500 ms. Bei dem gezeigten Ausführungsbeispiel zeigt die Kurve 166 weiterhin eine dritte Phase bzw. einen dritten Zeitraum 172, in dem an das Heizelement 148 eine elektrische Spannung angelegt ist und das Heizelement 148 somit angeschaltet ist, und eine vierte Phase bzw. einen vierten Zeitraum 174, in dem an das Heizelement 148 keine elektrische Spannung angelegt ist und das Heizelement 148 somit ausgeschaltet ist. Dabei folgt der vierte Zeitraum 174 auf den dritten Zeitraum 172. Eine Summe aus drittem Zeitraum 172 und viertem Zeitraum 174 ist beispielsweise 500 ms.

Zur Durchführung einer Diagnose der vierten elektrischen Leitung 146, die den zweiten gesonderten Anschluss P2 mit der Pumpzelle 140 bzw. der Gegenelektrode 144 verbindet, wird mittels des Steuergeräts 122 eine Spannungsanregung der zweiten Pumpzelle 140 zum Erzeugen eines Messsignals an dem Messwiderstand 160 durchgeführt. So wird an die zweite Pumpzelle 140 eine vorbestimmte elektrische Spannung 176 über den gemeinsamen Anschluss COM angelegt. Es wird eine Spannungsanregung der zweiten Pumpzelle 140 mittels der Anregungssignalquelle 156 durchgeführt. Die Spannungsanregung umfasst eine periodische Veränderung der vorbestimmten elektrischen Spannung. Dies erzeugt ein Messsignal 182 an dem Messwiderstand 160. Die Periodendauer ist dabei kleiner als eine elektrochemische Zeitkonstante der zweiten Pumpzelle 140. Das Messsignal 182 am Messwiderstand 160 zeigt daher eine Überlagerung des eigentlichen Messsignals mit der periodischen Spannungsanregung. Das Messsignal 182 wird mittels eines nicht näher gezeigten Tiefpassfilters des Steuergeräts 122 gefiltert und über eine Schnittstelle des Steuergerätes an ein Motorsteuergerät übertragen. Die vorbestimmte elektrische Spannung wird mit einer Frequenz verändert, die größer als die Bandbreite des Tiefpassfilters ist. Nach Tiefpassfilterung des Messsignals 182 ergibt sich das Signal 198, das von der periodischen Spannungsanregung bereinigt ist. Somit ist das Messsignal für NOx nicht verfälscht. Die vierte elektrisch leitende Verbindung 146 wird als intakt identifiziert, falls das Messsignal 182 vor Tiefpassfilterung, d.h. das Rohsignal, eine periodische Veränderung 200 zeigt, und wird als defekt identifiziert, falls das Messsignal 182 vor Tiefpassfilterung keine periodische Veränderung 200 zeigt. Eine solche periodische Veränderung wird von der Frequenz der Spannungsänderung bei intakter elektrischer Verbindung verursacht. Bei dem gezeigten Ausführungsbeispiel liegt in dem ersten Zeitraum 168 eine periodische Veränderung 200 vor, wohingegen in dem dritten Zeitraum 172 keine periodische Veränderung 200 vorliegt. Dies weist darauf hin, dass in dem dritten Zeitraum 172 eine Unterbrechung der vierten elektrischen Leitung 146 vorliegt. Die Frequenz auf der vierten elektrischen Leitung 146 muss nicht zwingend über den gesamten Zeitraum angeregt werden. Das Anlegung der höherfrequenten Spannungsänderung im Bereich um nahezu 0 ppm NOx reicht für die elektrische Diagnose aus.

## Patentansprüche

1. Verfahren zum Betreiben eines Sensors (100) zum Nachweis mindestens eines Anteils einer Messgaskomponente mit gebundenem Sauerstoff in einem Messgas, insbesondere in einem Abgas einer Verbrennungskraftmaschine, umfassend ein Sensorelement (110), wobei das Sensorelement (110) eine erste Pumpzelle (112), die eine äußere Pumpelektrode (114) und eine innere Pumpelektrode (116) aufweist und die an einem ersten Hohlraum (126) anliegt, welcher mit dem Messgas in Verbindung steht, eine Referenzzelle (130), welche eine Nernst-Elektrode (132) und eine Referenzelektrode (134) aufweist und die an einem Referenzgasraum (138) anliegt, und eine zweite Pumpzelle (140), die eine Pumpelektrode (142) und eine Gegenelektrode (144) aufweist und die an einem zweiten Hohlraum (145) anliegt, wobei sich die Gegenelektrode (144) der zweiten Pumpzelle (140) im Referenzgasraum (138) befindet, wobei ein elektronisches Steuergerät (122), das zumindest über einen ersten gesonderten Anschluss (P1) für die erste Pumpzelle (112) und einen zweiten gesonderten Anschluss (P2) für die zweite Pumpzelle (140) verfügt, mit dem Sensorelement (110) verbunden wird, wobei die erste Pumpzelle (112) mittels einer elektrisch leitenden Verbindung (120) mit dem ersten gesonderten Anschluss (P1) verbunden wird, wobei die zweite Pumpzelle (140) mittels einer elektrisch leitenden Verbindung (146) mit dem zweiten gesonderten Anschluss (P2) verbunden wird, wobei in der elektrisch leitenden Verbindung (146), die die zweite Pumpzelle (140) mit dem zweiten gesonderten Anschluss (P2) verbindet, ein Messwiderstand (160) vorgesehen wird, wobei mittels des Steuergeräts (122) eine Strom- und/oder Spannungsanregung der zweiten Pumpzelle (140) zum Erzeugen eines Messsignals (182) an dem Messwiderstand (160) durchgeführt wird, wobei an die zweite Pumpzelle (140) eine vorbestimmte elektrische Spannung (U_{P2}) angelegt wird, wobei eine Spannungsanregung der zweiten Pumpzelle (140) durchgeführt wird, wobei für eine erste vorbestimmte Zeit (178) die vorbestimmte elektrische Spannung (U_{P2}) erhöht wird und für eine zweite vorbestimmte Zeit (180) die vorbestimmte elektrische Spannung (U_{P2}) erniedrigt wird, wobei ein Integral der angelegten elektrischen Spannung für die erste vorbestimmte Zeit (178) und die zweite vorbestimmte Zeit (180) einen Wert von Null aufweist, wobei die elektrisch leitende Verbindung (146), die die zweite Pumpzelle (140) mit dem zweiten gesonderten Anschluss (P2) verbindet, als intakt identifiziert wird, falls das Messsignal (182) für die erste vorbestimmte Zeit (178) und die zweite vorbestimmte Zeit (180) eine Wert von ungleich Null aufweist, und als defekt identifiziert wird, falls das Messsignal (182) für die erste vorbestimmte Zeit (178) und die zweite vorbestimmte Zeit (180) einen Wert von Null aufweist.

2. Verfahren zum Betreiben eines Sensors (100) zum Nachweis mindestens eines Anteils einer Messgaskomponente mit gebundenem Sauerstoff in einem Messgas, insbesondere in einem Abgas einer Verbrennungskraftmaschine, umfassend ein Sensorelement (110), wobei das Sensorelement (110) eine erste Pumpzelle (112), die eine äußere Pumpelektrode (114) und eine innere Pumpelektrode (116) aufweist und die an einem ersten Hohlraum (126) anliegt, welcher mit dem Messgas in Verbindung steht, eine Referenzzelle (130), welche eine Nernst-Elektrode (132) und eine Referenzelektrode (134) aufweist und die an einem Referenzgasraum (138) anliegt, und eine zweite Pumpzelle (140), die eine Pumpelektrode (142) und eine Gegenelektrode (144) aufweist und die an einem zweiten Hohlraum (145) anliegt, wobei sich die Gegenelektrode (144) der zweiten Pumpzelle (140) im Referenzgasraum (138) befindet, wobei ein elektronisches Steuergerät (122), das zumindest über einen ersten gesonderten Anschluss (P1) für die erste Pumpzelle (112) und einen zweiten gesonderten Anschluss (P2) für die zweite Pumpzelle (140) verfügt, mit dem Sensorelement (110) verbunden wird, wobei die erste Pumpzelle (112) mittels einer elektrisch leitenden Verbindung (120) mit dem ersten gesonderten Anschluss (P1) verbunden wird, wobei die zweite Pumpzelle (140) mittels einer elektrisch leitenden Verbindung (146) mit dem zweiten gesonderten Anschluss (P2) verbunden wird, wobei in der elektrisch leitenden Verbindung (146), die die zweite Pumpzelle (140) mit dem zweiten gesonderten Anschluss (P2) verbindet, ein Messwiderstand (160) vorgesehen wird, wobei mittels des Steuergeräts (122) eine Strom- und/oder Spannungsanregung der zweiten Pumpzelle (140) zum Erzeugen eines Messsignals (182) an dem Messwiderstand (160) durchgeführt wird, wobei in die zweite Pumpzelle (140) ein vorbestimmter elektrischer Strom eingeprägt wird, wobei eine Stromanregung der zweiten Pumpzelle (140) durchgeführt wird, wobei für eine erste vorbestimmte Zeit (178) der vorbestimmte elektrische Strom erhöht wird und für eine zweite vorbestimmte Zeit (180) der vorbestimmte elektrische Strom erniedrigt wird, wobei die erste vorbestimmte Zeit (178) und die zweite vorbestimmte Zeit (180) identisch lang sind, wobei die elektrisch leitende Verbindung (146), die die zweite Pumpzelle (140) mit dem zweiten gesonderten Anschluss (P2) verbindet, als intakt identifiziert wird, falls eine an die zweite Pumpzelle (140) angelegte elektrische Spannung (U_{P2}) für die erste vorbestimmte Zeit (178) und für die zweite vorbestimmte Zeit (180) einen Schwellwert (192) unterschreitet, und als defekt identifiziert wird, falls eine an die zweite Pumpzelle (140) angelegte elektrische Spannung (U_{P2}) für die erste vorbestimmte Zeit (178) und für die zweite vorbestimmte Zeit (180) einen Schwellwert (192) überschreitet.

3. Verfahren zum Betreiben eines Sensors (100) zum Nachweis mindestens eines Anteils einer Messgaskomponente mit gebundenem Sauerstoff in einem Messgas, insbesondere in einem Abgas einer Verbrennungskraftmaschine, umfassend ein Sensorelement (110), wobei das Sensorelement (110) eine erste Pumpzelle (112), die eine äußere Pumpelektrode (114) und eine innere Pumpelektrode (116) aufweist und die an einem ersten Hohlraum (126) anliegt, welcher mit dem Messgas in Verbindung steht, eine Referenzzelle (130), welche eine Nernst-Elektrode (132) und eine Referenzelektrode (134) aufweist und die an einem Referenzgasraum (138) anliegt, und eine zweite Pumpzelle (140), die eine Pumpelektrode (142) und eine Gegenelektrode (144) aufweist und die an einem zweiten Hohlraum (145) anliegt, wobei sich die Gegenelektrode (144) der zweiten Pumpzelle (140) im Referenzgasraum (138) befindet, wobei ein elektronisches Steuergerät (122), das zumindest über einen ersten gesonderten Anschluss (P1) für die erste Pumpzelle (112) und einen zweiten gesonderten Anschluss (P2) für die zweite Pumpzelle (140) verfügt, mit dem Sensorelement (110) verbunden wird, wobei die erste Pumpzelle (112) mittels einer elektrisch leitenden Verbindung (120) mit dem ersten gesonderten Anschluss (P1) verbunden wird, wobei die zweite Pumpzelle (140) mittels einer elektrisch leitenden Verbindung (146) mit dem zweiten gesonderten Anschluss (P2) verbunden wird, wobei in der elektrisch leitenden Verbindung (146), die die zweite Pumpzelle (140) mit dem zweiten gesonderten Anschluss (P2) verbindet, ein Messwiderstand (160) vorgesehen wird, wobei mittels des Steuergeräts (122) eine Strom- und/oder Spannungsanregung der zweiten Pumpzelle (140) zum Erzeugen eines Messsignals (182) an dem Messwiderstand (160) durchgeführt wird, wobei an die zweite Pumpzelle (140) eine vorbestimmte elektrische Spannung (U_{P2}) angelegt wird, wobei eine Spannungsanregung der zweiten Pumpzelle (140) durchgeführt wird, wobei die Spannungsanregung eine periodische Veränderung der vorbestimmten elektrischen Spannung (U_{P2}) umfasst, wobei die elektrisch leitende Verbindung (146), die die zweite Pumpzelle (140) mit dem zweiten gesonderten Anschluss (P2) verbindet, als intakt identifiziert wird, falls das Messsignal (182) eine periodische Veränderung (200) zeigt, und als defekt identifiziert wird, falls das Messsignal (182) keine periodische Veränderung (200) zeigt..

4. Verfahren nach Anspruch 3, wobei die Periodendauer kleiner als eine elektrochemische Zeitkonstante der zweiten Pumpzelle (140) ist.

5. Verfahren nach Anspruch 3 oder 4, wobei das Messsignal (182) mittels eines Tiefpassfilters gefiltert wird, wobei die vorbestimmte elektrische Spannung (U_{P2}) mit einer Frequenz verändert wird, die größer als die Bandbreite des Tiefpassfilters ist.

6. Elektronisches Steuergerät, welches über einen ersten gesonderten Anschluss (P1), einen zweiten gesonderten Anschluss (P2) verfügt, und über Mittel, die geeignet sind, die Schritte des Verfahrens nach einem der vorangehenden Ansprüchen auszuführen.

7. Computerprogramm, umfassend Befehle, die bewirken, dass ein Steuergerät nach Anspruch 6, die Schritte des Verfahrens nach einem der Ansprüche 1 bis 5 ausführt.

8. Elektronisches Speichermedium, auf welchem ein Computerprogramm nach dem vorhergehenden Anspruch gespeichert ist.

## Claims

1. Method for operating a sensor (100) for detecting at least one portion of a measurement gas component with bound oxygen in a measurement gas, in particular in an exhaust gas of an internal combustion engine, comprising a sensor element (110), wherein the sensor element (110) has a first pump cell (112) which has an outer pump electrode (114) and an inner pump electrode (116) and is present at a first cavity (126) connected to the measurement gas, a reference cell (130) which has a Nernst electrode (132) and a reference electrode (134) and is present at a reference gas space (138), and a second pump cell (140) which has a pump electrode (142) and a counter-electrode (144) and is present at a second cavity (145), wherein the counter-electrode (144) of the second pump cell (140) is in the reference gas space (138), wherein an electronic control device (122), which has at least a first separate terminal (P1) for the first pump cell (112) and a second separate terminal (P2) for the second pump cell (140), is connected to the sensor element (110), wherein the first pump cell (112) is connected to the first separate terminal (P1) by means of an electrically conductive connection (120), wherein the second pump cell (140) is connected to the second separate terminal (P2) by means of an electrically conductive connection (146), wherein a measurement resistor (160) is provided in the electrically conductive connection (146) which connects the second pump cell (140) to the second separate terminal (P2), wherein the control device (122) is used to carry out a current and/or voltage excitation of the second pump cell (140) in order to generate a measurement signal (182) at the measurement resistor (160), wherein a predetermined electrical voltage (U_{P2}) is applied to the second pump cell (140), wherein a voltage excitation of the second pump cell (140) is carried out, wherein the predetermined electrical voltage (U_{P2}) is increased for a first predetermined time (178) and the predetermined electrical voltage (U_{P2}) is reduced for a second predetermined time (180), wherein an integral of the applied electrical voltage has a value of zero for the first predetermined time (178) and the second predetermined time (180), wherein the electrically conductive connection (146) which connects the second pump cell (140) to the second separate terminal (P2) is identified as intact if the measurement signal (182) has a value not equal to zero for the first predetermined time (178) and the second predetermined time (180) and is identified as defective if the measurement signal (182) has a value of zero for the first predetermined time (178) and the second predetermined time (180).

2. Method for operating a sensor (100) for detecting at least one portion of a measurement gas component with bound oxygen in a measurement gas, in particular in an exhaust gas of an internal combustion engine, comprising a sensor element (110), wherein the sensor element (110) has a first pump cell (112) which has an outer pump electrode (114) and an inner pump electrode (116) and is present at a first cavity (126) connected to the measurement gas, a reference cell (130) which has a Nernst electrode (132) and a reference electrode (134) and is present at a reference gas space (138), and a second pump cell (140) which has a pump electrode (142) and a counter-electrode (144) and is present at a second cavity (145), wherein the counter-electrode (144) of the second pump cell (140) is in the reference gas space (138), wherein an electronic control device (122), which has at least a first separate terminal (P1) for the first pump cell (112) and a second separate terminal (P2) for the second pump cell (140), is connected to the sensor element (110), wherein the first pump cell (112) is connected to the first separate terminal (P1) by means of an electrically conductive connection (120), wherein the second pump cell (140) is connected to the second separate terminal (P2) by means of an electrically conductive connection (146), wherein a measurement resistor (160) is provided in the electrically conductive connection (146) which connects the second pump cell (140) to the second separate terminal (P2), wherein the control device (122) is used to carry out a current and/or voltage excitation of the second pump cell (140) in order to generate a measurement signal (182) at the measurement resistor (160), wherein a predetermined electrical current is injected into the second pump cell (140), wherein a current excitation of the second pump cell (140) is carried out, wherein the predetermined electrical current is increased for a first predetermined time (178) and the predetermined electrical current is reduced for a second predetermined time (180), wherein the first predetermined time (178) and the second predetermined time (180) have an identical length, wherein the electrically conductive connection (146) which connects the second pump cell (140) to the second separate terminal (P2) is identified as intact if an electrical voltage (U_{P2}) applied to the second pump cell (140) falls below a threshold value (192) for the first predetermined time (178) and the second predetermined time (180) and is identified as defective if an electrical voltage (U_{P2}) applied to the second pump cell (140) exceeds a threshold value (192) for the first predetermined time (178) and the second predetermined time (180).

3. Method for operating a sensor (100) for detecting at least one portion of a measurement gas component with bound oxygen in a measurement gas, in particular in an exhaust gas of an internal combustion engine, comprising a sensor element (110), wherein the sensor element (110) has a first pump cell (112) which has an outer pump electrode (114) and an inner pump electrode (116) and is present at a first cavity (126) connected to the measurement gas, a reference cell (130) which has a Nernst electrode (132) and a reference electrode (134) and is present at a reference gas space (138), and a second pump cell (140) which has a pump electrode (142) and a counter-electrode (144) and is present at a second cavity (145), wherein the counter-electrode (144) of the second pump cell (140) is in the reference gas space (138), wherein an electronic control device (122), which has at least a first separate terminal (P1) for the first pump cell (112) and a second separate terminal (P2) for the second pump cell (140), is connected to the sensor element (110), wherein the first pump cell (112) is connected to the first separate terminal (P1) by means of an electrically conductive connection (120), wherein the second pump cell (140) is connected to the second separate terminal (P2) by means of an electrically conductive connection (146), wherein a measurement resistor (160) is provided in the electrically conductive connection (146) which connects the second pump cell (140) to the second separate terminal (P2), wherein the control device (122) is used to carry out a current and/or voltage excitation of the second pump cell (140) in order to generate a measurement signal (182) at the measurement resistor (160), wherein a predetermined electrical voltage (U_{P2}) is applied to the second pump cell (140), wherein a voltage excitation of the second pump cell (140) is carried out, wherein the voltage excitation comprises a periodic change in the predetermined electrical voltage (U_{P2}), wherein the electrically conductive connection (146) which connects the second pump cell (140) to the second separate terminal (P2) is identified as intact if the measurement signal (182) exhibits a periodic change (200) and is identified as defective if the measurement signal (182) does not exhibit a periodic change (200).

4. Method according to Claim 3, wherein the period duration is less than an electrochemical time constant of the second pump cell (140).

5. Method according to Claim 3 or 4, wherein the measurement signal (182) is filtered by means of a low-pass filter, wherein the predetermined electrical voltage (U_{P2}) is changed at a frequency which is greater than the bandwidth of the low-pass filter.

6. Electronic control device having a first separate terminal (P1), a second separate terminal (P2) and means which are suitable for carrying out the steps of the method according to one of the preceding claims.

7. Computer program comprising instructions which cause a control device according to Claim 6 to carry out the steps of the method according to one of Claims 1 to 5.

8. Electronic storage medium, on which a computer program according to the preceding claim is stored.

## Revendications

1. Procédé permettant de faire fonctionner un capteur (100) pour détecter au moins une fraction d'un composant de gaz de mesure avec de l'oxygène lié dans un gaz de mesure, en particulier dans un gaz d'échappement d'un moteur à combustion interne, comprenant un élément capteur (110), l'élément capteur (110) présentant une première cellule de pompage (112) qui présente une électrode de pompage extérieure (114) et une électrode de pompage intérieure (116) et qui est adjacente à une première cavité (126) qui est en communication avec le gaz de mesure, une cellule de référence (130) qui présente une électrode de Nernst (132) et une électrode de référence (134) et qui est adjacente à une chambre à gaz de référence (138), et une deuxième cellule de pompage (140) qui présente une électrode de pompage (142) et une contre-électrode (144) qui est adjacente à une deuxième cavité (145), dans lequel la contre-électrode (144) de la deuxième cellule de pompage (140) se trouve dans la chambre à gaz de référence (138), dans lequel un appareil de commande électronique (122) qui dispose au moins d'une première borne séparée (P1) pour la première cellule de pompage (112) et d'une deuxième borne séparée (P2) pour la deuxième cellule de pompage (140) est relié à l'élément capteur (110), dans lequel la première cellule de pompage (112) est reliée à la première borne séparée (P1) au moyen d'une liaison électriquement conductrice (120), dans lequel la deuxième cellule de pompage (140) est reliée à la deuxième borne séparée (P2) au moyen d'une liaison électriquement conductrice (146), dans lequel une résistance de mesure (160) est prévue dans la liaison électriquement conductrice (146) qui relie la deuxième cellule de pompage (140) à la deuxième borne séparée (P2), dans lequel une excitation de courant et/ou de tension de la deuxième cellule de pompage (140) est effectuée au moyen de l'appareil de commande (122) pour générer un signal de mesure (182) au niveau de la résistance de mesure (160), dans lequel une tension électrique prédéterminée (Uₚ₂) est appliquée à la deuxième cellule de pompage (140), dans lequel une excitation de tension de la deuxième cellule de pompage (140) est effectuée, dans lequel la tension électrique prédéterminée (Uₚ₂) est augmentée pendant un premier délai prédéterminé (178) et la tension électrique prédéterminée (Uₚ₂) est diminuée pendant un deuxième délai prédéterminé (180), dans lequel une intégrale de la tension électrique appliquée présente une valeur de zéro pendant le premier délai prédéterminé (178) et le deuxième délai prédéterminé (180), dans lequel la liaison électriquement conductrice (146) qui relie la deuxième cellule de pompage (140) à la deuxième borne séparée (P2) est identifiée comme étant intacte si le signal de mesure (182) pendant le premier délai prédéterminé (178) et le deuxième délai prédéterminé (180) présente une valeur différente de zéro, et est identifiée comme défectueuse si le signal de mesure (182) présente une valeur de zéro pendant le premier délai prédéterminé (178) et le deuxième délai prédéterminé (180).

2. Procédé permettant de faire fonctionner un capteur (100) pour détecter au moins une fraction d'un composant de gaz de mesure avec de l'oxygène lié dans un gaz de mesure, en particulier dans un gaz d'échappement d'un moteur à combustion interne, comprenant un élément capteur (110), l'élément capteur (110) présentant une première cellule de pompage (112) qui présente une électrode de pompage extérieure (114) et une électrode de pompage intérieure (116) et qui est adjacente à une première cavité (126) qui est en communication avec le gaz de mesure, une cellule de référence (130) qui présente une électrode de Nernst (132) et une électrode de référence (134) et qui est adjacente à une chambre à gaz de référence (138), et une deuxième cellule de pompage (140) qui présente une électrode de pompage (142) et une contre-électrode (144) qui est adjacente à une deuxième cavité (145), dans lequel la contre-électrode (144) de la deuxième cellule de pompage (140) se trouve dans la chambre à gaz de référence (138), dans lequel un appareil de commande électronique (122) qui dispose au moins d'une première borne séparée (P1) pour la première cellule de pompage (112) et d'une deuxième borne séparée (P2) pour la deuxième cellule de pompage (140) est relié à l'élément capteur (110), dans lequel la première cellule de pompage (112) est reliée à la première borne séparée (P1) au moyen d'une liaison électriquement conductrice (120), dans lequel la deuxième cellule de pompage (140) est reliée à la deuxième borne séparée (P2) au moyen d'une liaison électriquement conductrice (146), dans lequel une résistance de mesure (160) est prévue dans la liaison électriquement conductrice (146) qui relie la deuxième cellule de pompage (140) à la deuxième borne séparée (P2), dans lequel une excitation de courant et/ou de tension de la deuxième cellule de pompage (140) est effectuée au moyen de l'appareil de commande (122) pour générer un signal de mesure (182) au niveau de la résistance de mesure (160), dans lequel un courant électrique prédéterminé est appliqué à la deuxième cellule de pompage (140), dans lequel une excitation de courant de la deuxième cellule de pompage (140) est effectuée, dans lequel le courant électrique prédéterminé est augmenté pendant un premier délai prédéterminé (178) et le courant électrique prédéterminé est diminué pendant un deuxième délai prédéterminé (180), dans lequel le premier délai prédéterminé (178) et le deuxième délai prédéterminé (180) sont de durée identique, dans lequel la liaison électriquement conductrice (146) qui relie la deuxième cellule de pompage (140) à la deuxième borne séparée (P2) est identifiée comme étant intacte si une tension électrique (Uₚ₂) appliquée à la deuxième cellule de pompage (140) soupasse une valeur seuil (192) pendant le premier délai prédéterminé (178) et pendant le deuxième délai prédéterminé (180), et est identifiée comme étant défectueuse si une tension électrique (Uₚ₂) appliquée à la deuxième cellule de pompage (140) dépasse une valeur seuil (192) pendant le premier délai prédéterminé (178) et pendant le deuxième délai prédéterminé (180).

3. Procédé permettant de faire fonctionner un capteur (100) pour détecter au moins une fraction d'un composant de gaz de mesure avec de l'oxygène lié dans un gaz de mesure, en particulier dans un gaz d'échappement d'un moteur à combustion interne, comprenant un élément capteur (110), l'élément capteur (110) présentant une première cellule de pompage (112) qui présente une électrode de pompage extérieure (114) et une électrode de pompage intérieure (116) et qui est adjacente à une première cavité (126) qui est en communication avec le gaz de mesure, une cellule de référence (130) qui présente une électrode de Nernst (132) et une électrode de référence (134) et qui est adjacente à une chambre à gaz de référence (138), et une deuxième cellule de pompage (140) qui présente une électrode de pompage (142) et une contre-électrode (144) qui est adjacente à une deuxième cavité (145), dans lequel la contre-électrode (144) de la deuxième cellule de pompage (140) se trouve dans la chambre à gaz de référence (138), dans lequel un appareil de commande électronique (122) qui dispose au moins d'une première borne séparée (P1) pour la première cellule de pompage (112) et d'une deuxième borne séparée (P2) pour la deuxième cellule de pompage (140) est relié à l'élément capteur (110), dans lequel la première cellule de pompage (112) est reliée à la première borne séparée (P1) au moyen d'une liaison électriquement conductrice (120), dans lequel la deuxième cellule de pompage (140) est reliée à la deuxième borne séparée (P2) au moyen d'une liaison électriquement conductrice (146), dans lequel une résistance de mesure (160) est prévue dans la liaison électriquement conductrice (146) qui relie la deuxième cellule de pompage (140) à la deuxième borne séparée (P2), dans lequel une excitation de courant et/ou de tension de la deuxième cellule de pompage (140) est effectuée au moyen de l'appareil de commande (122)pour générer un signal de mesure (182) au niveau de la résistance de mesure (160), dans lequel une tension électrique prédéterminée (Uₚ₂) est appliquée à la deuxième cellule de pompage (140), dans lequel une excitation de tension de la deuxième cellule de pompage (140) est effectuée, dans lequel l'excitation de tension comprend une variation périodique de la tension électrique prédéterminée (Uₚ₂), dans lequel la liaison électriquement conductrice (146) qui relie la deuxième cellule de pompage (140) à la deuxième borne séparée (P2) est identifiée comme étant intacte si le signal de mesure (182) indique une variation périodique (200), et est identifiée comme étant défectueuse si le signal de mesure (182) n'indique aucune variation périodique (200).

4. Procédé selon la revendication 3, dans lequel la durée de période est inférieure à une constante de temps électrochimique de la deuxième cellule de pompage (140).

5. Procédé selon la revendication 3 ou 4, dans lequel le signal de mesure (182) est filtré au moyen d'un filtre passe-bas, dans lequel la tension électrique prédéterminée (Uₚ₂) est modifiée à une fréquence qui est supérieure à la bande passante du filtre passe-bas.

6. Appareil de commande électronique qui dispose d'une première borne séparée (P1), d'une deuxième borne séparée (P2), et de moyens qui sont adaptés pour exécuter les étapes du procédé selon l'une quelconque des revendications précédentes.

7. Programme informatique, comprenant des instructions qui font qu'un appareil de commande selon la revendication 6 exécute les étapes du procédé selon l'une quelconque des revendications 1 à 5.

8. Support de stockage électronique sur lequel est stocké un programme informatique selon la revendication précédente.
